# EUROPEAN PATENT SPECIFICATION

(11) **EP 3 990 085 B1**
(45) Date of publication and mention of the grant of the patent: **30.08.2023**
(21) Application number: 20736569.3
(22) Date of filing: 25.06.2020
(51) Int. Cl.: A61M 25/00, A61B 5/20, A61B 5/00

(54) **CATHETER COMPRISING COMBINED VALVE AND SENSOR**
KATHETER MIT KOMBINIERTEM VENTIL UND SENSOR
CATHÉTER COMPRENANT UNE VALVE ET UN CAPTEUR COMBINÉS

(30) Priority: 26.06.2019 DK PA201970407
(43) Date of publication of application: 04.05.2022
(73) Proprietor: Coloplast A/S, 3050 Humlebaek (DK)
(72) Inventor: PEDERSEN, Troels Gottfried, 3250 Gilleleje (DK)
(86) International application number: PCT/DK2020/050193
(87) International publication number: WO 2020/259783

(56) References cited:
- GB-A- 2 395 128
- KR-A- 20140 007 693
- US-A- 6 102 888
- US-A1- 2005 256 447

## Description

### FIELD OF THE INVENTION

The present invention relates to relieving urinary retention and the field of intermittent catheterization.

### BRIEF SUMMARY

GB2395128 describes a catheter control device for a urinary catheter using a sensor to open and close a valve for drainage. KR 10 2014 0007693 describes a urination control device using a valve which can be controlled by magnetic forces. US 2003/0256447 describes an apparatus for controlling a catheter which comprises the use of valves and sensors which sense one or more properties of the liquid present in the apparatus. The valves are actuated in response to the sensed parameters. US 6 102 888 describes a urinary tract irrigating and draining assembly.

The invention is defined in the appended claims 1-14.

Embodiments provide an intermittent urinary catheterisation assembly comprising an intermittent urinary catheter comprising a connecting portion being integral with or mounted to the non-insertable portion. The intermittent urinary catheter further comprises a conduit extending longitudinally within the tube and defining at least part of a flow path from a distal insertion end of the catheter to a proximal outlet end thereof. The intermittent urinary catheterisation assembly further comprises a measuring system configured to measure at least a pressure in the conduit and/or in a space in communication with the conduit. The measuring system comprises at least one valve element in fluid communication with the conduit when the signal processing device is secured in relation to the intermittent urinary catheter. The at least one valve element comprises a cut-off element configured to switch from a closed state disabling liquid flow in the conduit to an open state enabling liquid flow in the conduit in response to a pressure applied to the cut-off element by liquid in the conduit. The measuring system further comprises at least one sensor element configured to measure a parameter indicative of the pressure applied to the cut-off element when the cut-off element switches from the closed state to the open state. The measuring system is configured to detect a change of state of the cut-off element from the closed state to the open state. In one aspect, the signal processing device comprises a housing with an engagement mechanism for detachably securing the signal processing device in relation to the connecting portion of the intermittent urinary catheter. In another aspect, the measuring system comprising the signal processing device is securely connected to or integrated with the non-insertable portion of the catheter tube.

### BRIEF DESCRIPTION OF THE DRAWINGS

The accompanying drawings are included to provide a further understanding of embodiments and are incorporated in and constitute a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figs. 1-3 illustrate schematic cross-sectional views of different embodiments of an intermittent urinary catheter assembly with an intermittent urinary catheter and a measuring system.
Fig. 4 illustrates a schematic cross-sectional view of an embodied intermittent urinary catheter.

### DETAILED DESCRIPTION

Intermittent catheters are typically inserted by the user him- or herself and sits only in the urethra and bladder for as long as it takes to empty the bladder - e.g. for about 5-10 minutes. Intermittent catheters are used every 4-6 hours to empty the bladder corresponding roughly to the interval that people having no urinary problems will usually go to the bathroom. Intermittent catheters are typically more rigid than indwelling catheters since they have to be inserted by the user him-/herself and since they do not need to sit in the urethra for days or weeks. An important feature for the intermittent catheter is to ease the insertion into the urethra. This is done by providing the intermittent catheter with a low frictious surface. Non-limiting examples of such are hydrophilic coated catheters which are subsequently wetted by a swelling media in order to produce a low friction surface, or oil or water-based gel which is applied to the catheter before insertion into the urethra.

Intermittent urinary catheters may be provided with a hydrophilic coating that needs to be wetted prior to use and thereby absorbs a considerable amount of liquid. Such a hydrophilic coating will provide a very lubricious surface that has very low-friction when the catheter is to be inserted. Hydrophilic coated catheters, where the coating absorbs a considerable amount of liquid for a low frictious surface (swelling degree >100%), will not be suitable for indwelling catheters, because the hydrophilic surface coating would stick inside the mucosa of the urethra if left inside the body for a longer period, due to the hydrophilic coating transforming from being highly lubricious when fully wetted to being adhesive when the hydration level of the coating is reduced.

This invention relates to intermittent urinary catheters that may be provided with a hydrophilic coating of the kind that is wetted prior to use to absorb a considerable amount of liquid and to provide a very lubricious surface.

Usually catheters used as urinary draining devices are from size 8 FR to size 18 FR. FR (or French size or Charriere (Ch)) is a standard gauge for catheters approximately corresponding to the outer circumference in mm. More accurately, the outer diameter of the catheter in mm corresponds to FR divided by 3. Thus 8 FR corresponds to a catheter with an outer diameter of 2.7 mm and 18 FR corresponds to a catheter with an outer diameter of 6 mm.

Intermittent urinary catheters typically range from CH 8 - CH 16.

Intermittent urinary catheters are predominantly used for self-catheterisation by the user.

Embodiments provide an intermittent urinary catheter assembly and an intermittent urinary catheter that enable measurement of a pressure for increased utility and convenience for the user. The intermittent urinary catheter assembly and intermittent urinary catheter provide a sensor element configured to measure a parameter indicative of the pressure.

In a first aspect, embodiments provide an intermittent urinary catheterisation assembly comprising:
- an intermittent urinary catheter comprising:
   ∘ a catheter tube comprising an insertable portion intended for insertion into a user's urethra, a non-insertable portion not intended for insertion into the user's urethra, and a connecting portion being integral with or mounted to the non-insertable portion; and
   o a conduit extending longitudinally within the tube and defining at least part of a flow path from a distal insertion end of the catheter to a proximal outlet end thereof;
- a measuring system configured to measure at least a pressure in the conduit and/or in a space in communication with the conduit, wherein the measuring system comprises:
   ∘ a signal processing device comprising a housing with an engagement mechanism for detachably securing the signal processing device in relation to the connecting portion of the catheter;
   ∘ at least one valve element in fluid communication with the conduit when the signal processing device is secured in relation to the intermittent urinary catheter, the at least one valve element comprising a cut-off element configured to switch from a closed state disabling liquid flow in the conduit to an open state enabling liquid flow in the conduit in response to a pressure applied to the cut-off element by liquid in the conduit; and
   ∘ at least one sensor element configured to measure a parameter indicative of the pressure applied to the cut-off element when the cut-off element switches from the closed state to the open state,
wherein the measuring system is configured to detect a change of state of the cut-off element from the closed state to the open state.

In a second aspect, embodiments provide an intermittent urinary catheterisation assembly comprising:
- an intermittent urinary catheter comprising:
   ∘ a catheter tube comprising an insertable portion intended for insertion into a user's urethra, a non-insertable portion not intended for insertion into the user's urethra; and
   o a conduit extending longitudinally within the tube and defining at least part of a flow path from a distal insertion end of the catheter to a proximal outlet end thereof;
- a measuring system securely connected to or integrated with the non-insertable portion of the catheter tube for determining at least a pressure in the conduit and/or in a space in communication with the conduit, wherein the measuring system comprises:
   o a signal processing device;
   o at least one valve element in fluid communication with the conduit, the at least one valve element comprising a cut-off element configured to switch from a closed state disabling liquid flow in the conduit to an open state enabling liquid flow in the conduit in response to a pressure applied to the cut-off element by liquid in the conduit; and
   ∘ at least one sensor element configured to measure a parameter indicative of the pressure applied to the cut-off element when the cut-off element switches from the closed state to the open state,
wherein the measuring system is configured to detect if the cut-off element changes from the closed state to the open state.

Thanks to the provision of the measuring system comprising the at least one valve element and the at least one sensor element, a user of an embodied intermittent urinary catheter assembly is enabled to determine at least a pressure in the conduit and/or in a space in communication with the conduit. In embodiments, the measuring system determines the pressure in the conduit and/or in a space in communication with the conduit on the basis of the measured parameter.

The connecting portion of the intermittent urinary catheter in conjunction with the engagement mechanism in embodiments of the first aspect enables detachable securement of the signal processing device in relation to the catheter tube. Accordingly, the intermittent urinary catheter may be disposed of after each intermittent catheterisation, while the signal processing device may be used a plurality of times for more effective use of resources. In embodiments of the first aspect, when the connecting portion and the engagement mechanism are properly secured in relation to each other, fluid communication between the conduit and the at least one valve element is enabled.

The connecting portion of the catheter tube and the engagement mechanism of the measuring device in embodiments of the first aspect may comprise respective, mutually mating connection elements. In one illustrative example, the engagement mechanism comprises resilient members configured to engage and at least partially surround, e.g., grooves at the outer circumference of the connecting portion of the catheter tube, which may provide improved tactile feedback for, e.g., physically impaired users. The engagement mechanism and connecting portion may also be embodied as, e.g., counterparts of a detachable click-lock for ease of manufacture, mutually attracting magnets for ease of attachment due to the attractive forces of the magnets working at a distance, or a frictional coupling where a protruding connecting portion of the catheter tube is forced into a receiving portion of the engagement mechanism or vice versa.

Thanks to the provision of the measuring system being securely connected to or integrated with the non-insertable portion of the catheter tube in embodiments of the second aspect, a user is able to determine the at least one fluid parameter without establishment of a connection between the catheter tube and measuring system prior to use.

In present context, the terms 'upstream' and 'downstream' refer to the flow path having a direction from the distal insertion end of the intermittent urinary catheter towards the proximal outlet end of the intermittent urinary catheter. These terms apply analogously to extensions of the flow path in fluid communication with the flow path. Accordingly, upstream refers to a flow direction towards the distal insertion end of the intermittent urinary catheter and downstream refers to a flow direction away from the distal insertion end of the intermittent urinary catheter.

When the cut-off element is in the closed state and the intermittent urinary catheter and the signal processing device are connected, liquid flow in the conduit will be disabled and the pressure within the conduit and upstream of the cut-off element will become constant. Accordingly, the pressure applied to the cut-off element will correspond to the pressure in the conduit and/or a space in communication with the conduit such as the bladder. This allows the signal processing device to determine the pressure in the conduit and/or in a space in communication with the conduit on the basis of the measured parameter.

In embodiments, the measuring system is configured to change a state of the cut-off element from the open state to the closed state after a predetermined amount of time after detection of the change of state of the cut-off element from the closed state to the open state. This allows for repeated measurements of the pressure in the conduit and/or in a space in communication with the conduit.

Following paragraph is not claimed and for illustrative purposes: In embodiments, at least one of the at least one sensor element is comprised in the signal processing device. This allows the at least one sensor element in the signal processing device to be re-used while the intermittent urinary catheter according to the first aspect is conveniently disposed of. In one embodiment, the signal processing device comprises wired or wireless connection means for connecting to- and transmitting data to another electronic device such as, e.g., a portable electronic device such as a smartphone or laptop, or a stationary computer or server. In one embodiment, the signal processing device comprises data output means as those described above.

Intermittent urinary catheters are typically disposable.

In embodiments, the intermittent urinary catheter assembly further comprises an air outlet upstream of the at least one valve element, the air outlet being configured not to allow liquid passage. The air outlet enables air to escape from the conduit and liquid propagation towards the at least one valve element in its closed state is improved until the liquid reaches the at least one valve element in the closed state. This will make the application of pressure from the liquid in the conduit to the at least one valve element more direct, and a greater accuracy of measurement may be achieved. In embodiments, the air outlet is integrated in the at least one valve element, the at least one valve element in these embodiments allowing air but not liquid to pass in the closed state.

In embodiments, the at least one valve element is configured and controllable to:
∘ apply a force to the cut-off element to disable a liquid flow in the conduit when the signal processing device is secured in relation to the intermittent urinary catheter; and
∘ gradually decrease the force applied to the cut-off element,
and wherein the parameter indicative of the pressure applied to the cut-off element when the cut-off element switches from the closed state to the open state measurable by the at least one sensor element is indicative of the force applied to the cut-off element. The force applied by the pressure of the liquid in the conduit to the cut-off element will be equal to or slightly higher than the force applied to the cut-off element by the at least one valve element at the time of switching. Accordingly, the parameter indicative of the force applied to the cut-off element can readily be applied to determine a pressure in the conduit and/or in a space in communication with the conduit. Further, the parameter indicative of the force applied to the cut-off element may be straight-forward to determine as this could conveniently be a control parameter of the signal processing device.

In embodiments, at least one of the at least one sensor element is configured to measure the presence of liquid downstream of the at least one valve element for reliable detection of the cut-off element changing from the closed state to the open state.

In embodiments, the cut-off element comprises a ferromagnetic valve member and at least one electromagnet, the at least one electromagnet being configured to apply a variable force to the ferromagnetic valve member. Such embodiments allow for precise and remote variation of the force applied to the cut-off element by the at least one valve element.

In embodiments, the at least one sensor element is configured to measure an amount of electrical current conducted through the electromagnet. This allows precise measurement of the parameter indicative of the force applied to the cut-off element. The measurement may be performed indirectly in the sense of applying a known current through the electromagnet. The measurement may also be performed directly to allow measurement of, e.g., a current generated in the electromagnet caused by movement of the ferromagnetic valve member relative to the electromagnet. Such movement happen when the cut-off element switches from the closed state to the open state, and the measuring system is able to detect that the change of state of the cut-off element on the basis of the measured amount of current through the electromagnet.

In embodiments, the measuring system is configured to determine a fluid flow rate on the basis of at least the measured amount of current conducted through the electromagnet. This is enabled by correlating different vibrational modes of the ferromagnetic valve member to different fluid flow rates. The different vibrational modes may lead to measurable differences in the amount and rate of current conducted through the electromagnet on the basis of which the fluid flow rate is determined.

In embodiments, the measuring system is configured to detect said change of state of the cut-off element when the parameter or a derivate thereof exceeds a predetermined threshold value for simple detection of change of states.

In embodiments:
- the valve element further comprises a restriction element upstream of the cut-off member;
- the cut-off member is displaceable relative to the restriction element;
- the cut-off element is configured to sealably connect to the restriction element in the closed state of the valve element. Accordingly, the closed state is achievable by applying a force to the cut-off element in the direction of the restriction element. The pressure applied from liquid at least in fluid communication with the conduit then pushes the cut-off element away from the restriction element.

In embodiments:
- the at least one valve element further comprises a retention element downstream of the restriction element; wherein the retention element is configured to retain the cut-off element in the open state of the valve element. Accordingly, the cut-off element will be retained even in the open state and may be forced against the restriction element again for a further measurement.

In embodiments, the intermittent urinary catheter is configured to rest in the user's urethra for a period of time not exceeding 15 minutes. This allows the intermittent urinary catheter to comprise, e.g., coatings that do not allow extended duration of catheterisation required for indwelling catheterisation.

In embodiments, at least an outer surface of the insertable portion of the catheter tube comprises a hydrophilic surface coating. Such hydrophilic coating allows particularly convenient intermittent urinary catheterisation. In embodiments, the intermittent urinary catheterisation assembly comprises a package assembly, the package assembly enclosing the catheter tube and signal processing device and comprising an amount of liquid swelling medium for activation of the hydrophilic surface to provide the intermittent urinary catheter assembly in a ready-to-use state when the package assembly is in a storage state.

The hydrophilic coating may be provided only on the insertable part of the catheter. The hydrophilic surface coating is of the kind which, when hydrated or swelled using a swelling medium, reduces the friction on the surface area of the catheter which is intended to be inserted into the urinary channel of a user corresponding to the insertable part of the catheter.

An intermittent hydrophilic catheter differs from an indwelling catheter also in that the hydrophilic surface coating of such a catheter is not suitable for indwelling use, because the surface coating tends to stick inside the mucosa of the urethra if left inside the body for a period exceeding 5-20 minutes, due to the hydrophilic coating transforming from being highly lubricious when fully wetted (95% weight water) to being adhesive when the hydration level of the coating is reduced (<75% weight water).

In embodiments, the catheter tube is a single-lumen tube, of which the conduit constitutes the sole passage between the distal insertion end and the proximal outlet end of the intermittent urinary catheter. Such single-lumen tube catheters are generally not applicable for indwelling use because of the lack of a passage for conducting air to the balloon of an indwelling or Foley urinary catheter.

In embodiments, the signal processing device is configured to determine a fluid flow rate in the conduit on the basis of a measurement performed by the at least one sensor element.

Following paragraph is not claimed and for illustrative purposes:

In embodiments, at least the conduit of the intermittent urinary catheter has a pre-defined characteristic pressure drop, wherein the signal processing device is configured to determine a fluid flow rate on the basis of at least the pre-defined characteristic pressure drop and the parameter indicative of the pressure applied to the cut-off element when the cut-off element switches from the closed state to the open state. The pre-defined characteristic pressure drop is utilised to determine the characteristic pressure drop/fluid flow rate dependency and allows the fluid flow rate to be determined. The pre-defined characteristic pressure drop may be pre-determined on the basis of a reference measurement on the embodied intermittent urinary catheter, or the embodied intermittent urinary catheter may be a standardised intermittent urinary catheter wherein the pre-defined characteristic pressure drop is based on a reference measurement on another standardised intermittent urinary catheter.

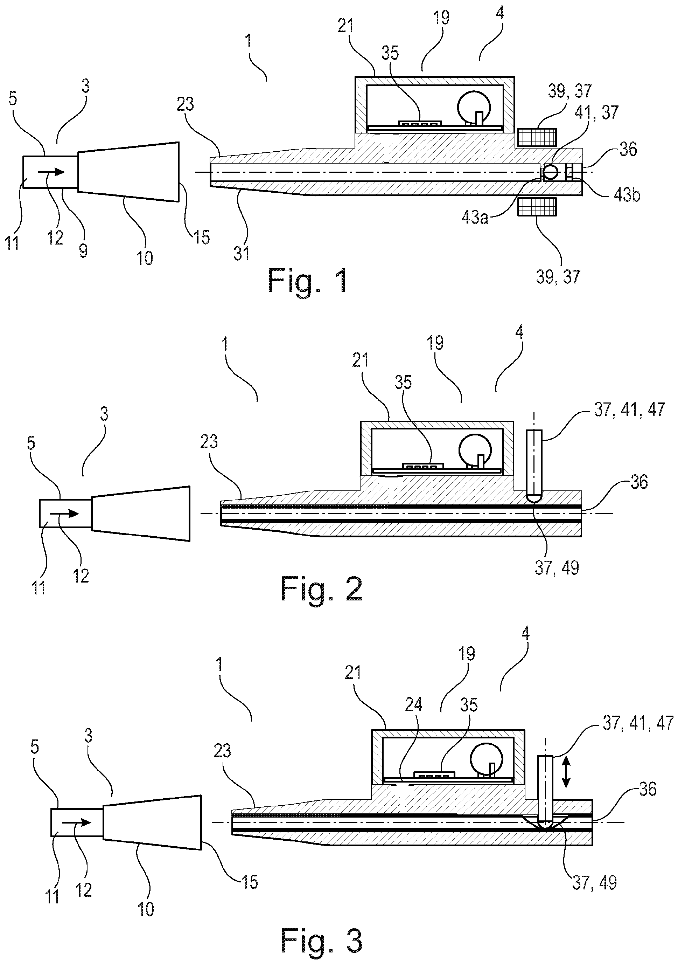

Fig. 1 illustrates a schematic cross-sectional view of an embodied intermittent urinary catheter assembly 1 with an intermittent urinary catheter 3 and a measuring system 4. The intermittent urinary catheter 3 comprises a catheter tube 5. The catheter tube 5 comprises an insertable portion 7 intended for insertion into a user's urethra, a non-insertable portion 9 not intended for insertion into the user's urethra, and a connecting portion 10 being integral with the non-insertable portion 9. The intermittent urinary catheter 3 further comprises a conduit 11 extending longitudinally within the catheter tube 5. The conduit 11 defines a flow path 12 from a distal insertion end 13 of the catheter 3 to a proximal outlet end 15 thereof. Only the non-insertable portion 9 of the catheter tube 5 and the connecting portion 10 and the proximal outlet end 15 of the intermittent urinary catheter 3 are illustrated, while the insertable portion 7 and the distal insertion end 13 of the catheter tube 5 extend outside the area illustrated in Fig. 1.

The intermittent urinary catheter assembly 1 of Fig. 1 further comprises a measuring system 4 for determining at least a pressure in the flow path 12 and/or in a space in communication with the flow path 12. In the illustrated embodiment, the measuring system 4 comprises a valve element 37 of an electronic type. An electromagnet comprising a coil 39 is wrapped around part of the signal processing device 19 to interact with a cut-off element 41 in the form of a ferromagnetic valve member 41, e.g. a ball, arranged between a restriction element 43a and a retention element 43b. By controlling the current in the coil 39, the ferromagnetic valve member 41 can be forced against the restriction element 43a in which case the valve 37 shuts off liquid flow, or it can be allowed to be pushed towards the retention element 43b which allows liquid to pass the valve element 37. The amount of current applied to the coil 39 is measured and gradually decreased to reduce the force applied to the ferromagnetic valve member 41 in the closed state of the cut-off member 41. When the ferromagnetic valve member 41 is pushed away from the retention element 43b by the pressure applied to it by liquid in communication with the conduit 5, the valve element 37 changes from a closed state (illustrated in Fig. 1) to an open state. The movement of the ferromagnetic valve member 41 relative to the coil 39 induces a measurable amount of current in the coil 39. The measuring system 4 detects the change of state from the closed state to the open state on the basis of a measurement of the induced current in the coil 39. The retention element 43b retains the ferromagnetic valve member 41 in the signal processing device 19, while allowing liquid to pass to a signal processing device outlet 36. The amount of current applied through the coil 39 by the measuring system 4 when the change of state is detected is measured and the measured amount of current is indicative of the pressure applied to the cut-off element 41 when the cut-off element 41 switches from the closed state to the open state.

The signal processing device 19 comprises a housing 21. The housing 21 has an engagement mechanism 23 that is able to detachably secure the signal processing device 19 in relation to the connecting portion 10 of the intermittent urinary catheter 3. In the embodiment of Fig. 1, the engagement mechanism 23 is detachably secured to the connecting portion 10 by firmly inserting the protruding engagement mechanism 23 in a receiving cavity of the connecting portion 10.

In one embodiment, the intermittent urinary catheter 3 is configured to rest in the user's urethra for a period of time not exceeding 15 minutes and at least an outer surface of the insertable portion 7 of the catheter tube 5 comprises a hydrophilic surface coating. The illustrated catheter tube 5 is a single-lumen tube, of which the conduit constitutes the sole passage between the distal insertion end 13 and the proximal outlet end 15.

In embodiments, the signal processing device 19 comprises a data processing unit 35 and a power source, such as a battery or photovoltaic system.

Figs. 2 and 3 illustrate a schematic cross-sectional view of an embodied intermittent urinary catheter assembly 1 with an intermittent urinary catheter 3 and a measuring system 4 being a variant of that of Fig. 1. In Figs. 2 and 3 the valve element 37 is in the form of a pushing rod 47 constituting the cut-off member 41 in combination with a flexible valve membrane 49 allowing the valve element 37 to be in the open state with the flexible valve membrane 49 not restricting the flow as illustrated in Fig. 2. The flexible membrane 49 also allows the valve element 37 to be in the closed state by pushing down the pushing rod 47 to force the flexible valve membrane 49 to shut off liquid flow past the valve 37 as illustrated in Fig. 3. The pushing rod 47 is pushed down by an electric motor (not shown) driven by a continuously measured amount of current. The amount of current measured when the pushing rod 47 is pushed towards the open state by the pressure caused by liquid in communication with the conduit 5 is indicative of the pressure in the conduit 5. The pressure in the conduit is determined on the basis of the measured current. An electrical contact (not shown) is activated when the pushing rod 47 moves away from the closed state. The measuring system 4 detects a change from the closed state to the open state on the basis of the activation of the electrical contact.

Figs. 1-3 have been illustrated and described in relation to the first aspect. Variations of the illustrated embodiments, wherein the measuring system 4 is securely connected to or integrated with the non-insertable portion 9 of the catheter tube 5 are also envisioned as in embodiments of the fourth aspect. In these variations the connecting portion 10 and the engagement mechanism 23 are exchanged with a permanent secure connection where the connecting portion 10 and the engagement mechanism 23 would otherwise detachably connect, or the catheter tube 5 and the signal processing device 19 are integrally formed where the connecting portion 10 and the engagement mechanism 23 would otherwise detachably connect.

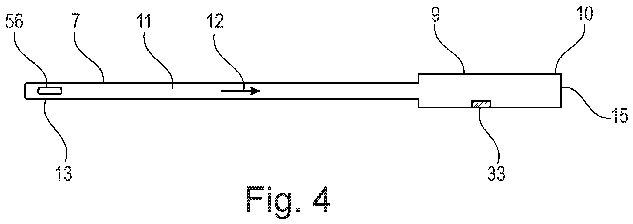

Fig. 4 illustrates a schematic cross-sectional view of an embodied intermittent urinary catheter 3. The intermittent urinary catheter 3 comprises a catheter tube 5. The catheter tube 5 comprises an insertable portion 7 intended for insertion into a user's urethra, a non-insertable portion 9 not intended for insertion into the user's urethra, and a connecting portion 10 being integral with the non-insertable portion 9. The intermittent urinary catheter 3 further comprises a conduit 11 extending longitudinally within the catheter tube 5. The conduit 11 defines a flow path 12 from a distal insertion end 13 of the catheter 3 to a proximal outlet end 15 thereof. The catheter tube 5 includes an eyelet 56 in the distal insertion end 13. The eyelet 56 allows liquid to enter the conduit 11.

## Claims

1. An intermittent urinary catheterisation assembly comprising:
- an intermittent urinary catheter comprising:
∘ a catheter tube comprising an insertable portion intended for insertion into a user's urethra, a non-insertable portion not intended for insertion into the user's urethra, and a connecting portion being integral with or mounted to the non-insertable portion; and
o a conduit extending longitudinally within the tube and defining at least part of a flow path from a distal insertion end of the catheter to a proximal outlet end thereof;
- a measuring system configured to measure at least a pressure in the conduit and/or in a space in communication with the conduit, wherein the measuring system comprises:
∘ a signal processing device comprising a housing with an engagement mechanism for detachably securing the signal processing device in relation to the connecting portion of the catheter;
∘ at least one valve element in fluid communication with the conduit when the signal processing device is secured in relation to the intermittent urinary catheter, the at least one valve element comprising a cut-off element configured to switch from a closed state disabling liquid flow in the conduit to an open state enabling liquid flow in the conduit in response to a pressure applied to the cut-off element by liquid in the conduit; and
∘ at least one sensor element configured to measure a parameter indicative of the pressure applied to the cut-off element when the cut-off element switches from the closed state to the open state,
wherein the measuring system is configured to detect a change of state of the cut-off element from the closed state to the open state.

2. An intermittent urinary catheterisation assembly according to claim 1, further comprising an air outlet upstream of the at least one valve element, the air outlet being configured not to allow liquid passage.

3. An intermittent urinary catheterisation assembly according to claim 1 or 2, wherein the at least one valve element is configured and controllable to:
∘ apply a force to the cut-off element to disable a liquid flow in the conduit when the signal processing device is secured in relation to the intermittent urinary catheter; and
∘ gradually decrease the force applied to the cut-off element,
and wherein the parameter indicative of the pressure applied to the cut-off element when the cut-off element switches from the closed state to the open state measurable by the at least one sensor element is indicative of the force applied to the cut-off element.

4. An intermittent urinary catheterisation assembly according to any of the preceding claims, wherein at least one of the at least one sensor element is configured to measure the presence of liquid downstream of the at least one valve element.

5. An intermittent urinary assembly according to any of the preceding claims, wherein the cut-off element comprises a ferromagnetic valve member and at least one electromagnet, the at least one electromagnet being configured to apply a variable force to the ferromagnetic valve member.

6. An intermittent urinary assembly according to claim 5, wherein the at least one sensor element is configured to measure an amount of electrical current conducted through the electromagnet.

7. An intermittent urinary catheterisation assembly according to any of the preceding claims, wherein the measuring system is configured to detect said change of state of the cut-off element when the parameter or a derivate thereof exceeds a predetermined threshold value.

8. An intermittent urinary catheter assembly according to any of the preceding claims, wherein:
- the valve element further comprises a restriction element upstream of the cut-off member;
- the cut-off member is displaceable relative to the restriction element;
- the cut-off element is configured to sealably connect to the restriction element in the closed state of the valve element.

9. An intermittent urinary catheter assembly according to claim 8, wherein:
- the at least one valve element further comprises a retention element downstream of the restriction element; wherein the retention element is configured to retain the cut-off element in the open state of the valve element.

10. An intermittent urinary catheterisation assembly according to any of the preceding claims, wherein the intermittent urinary catheter is configured to rest in the user's urethra for a period of time not exceeding 15 minutes.

11. An intermittent urinary catheterisation assembly according to any of the preceding claims, wherein at least an outer surface of the insertable portion of the catheter tube comprises a hydrophilic surface coating.

12. An intermittent urinary catheterisation assembly according to any of the preceding claims, wherein the catheter tube is a single-lumen tube, of which the conduit constitutes the sole passage between the distal insertion end and the proximal outlet end of the intermittent urinary catheter.

13. An intermittent urinary catheterisation assembly according to any of the preceding claims, wherein the signal processing device is configured to determine a fluid flow rate in the conduit on the basis of a measurement performed by the at least one sensor element.

14. An intermittent urinary catheterisation assembly comprising:
- an intermittent urinary catheter comprising:
∘ a catheter tube comprising an insertable portion intended for insertion into a user's urethra, a non-insertable portion not intended for insertion into the user's urethra; and
o a conduit extending longitudinally within the tube and defining at least part of a flow path from a distal insertion end of the catheter to a proximal outlet end thereof;
- a measuring system securely connected to or integrated with the non-insertable portion of the catheter tube for determining at least a pressure in the conduit and/or in a space in communication with the conduit, wherein the measuring system comprises:
o a signal processing device;
o at least one valve element in fluid communication with the conduit, the at least one valve element comprising a cut-off element configured to switch from a closed state disabling liquid flow in the conduit to an open state enabling liquid flow in the conduit in response to a pressure applied to the cut-off element by liquid in the conduit; and
∘ at least one sensor element configured to measure a parameter indicative of the pressure applied to the cut-off element when the cut-off element switches from the closed state to the open state,
wherein the measuring system is configured to detect if the cut-off element changes from the closed state to the open state.

## Patentansprüche

1. Intermittierende Harnkatheterisierungsanordnung, umfassend:
- einen intermittierenden Harnkatheter, umfassend:
o einen Katheterschlauch, der einen einführbaren Abschnitt, der zum Einsatz in die Harnröhre eines Benutzers gedacht ist, einen nicht einführbaren Abschnitt, der nicht zum Einsatz in die Harnröhre des Patienten gedacht ist, und einen Verbindungsabschnitt, der einstückig mit dem nicht einführbaren Abschnitt gebildet oder daran montiert ist, umfasst; und
o einen Kanal, der sich in Längsrichtung in dem Schlauch erstreckt und mindestens einen Teil eines Fließweges von einem distalen Einführende des Katheters zu einem proximalen Auslassende davon definiert;
- ein Messsystem, das dazu ausgelegt ist, mindestens einen Druck in dem Kanal und/oder in einem Raum in Verbindung mit dem Kanal zu messen, wobei das Messsystem umfasst:
o eine Signalverarbeitungseinrichtung, die ein Gehäuse mit einem Eingriffmechanismus zum lösbaren Befestigen der Signalverarbeitungseinrichtung in Bezug auf den Verbindungsabschnitt des Katheters umfasst;
o mindestens ein Ventilelement in Fluidverbindung mit dem Kanal, wenn die Signalverarbeitungseinrichtung in Bezug auf den intermittierenden Harnkatheter befestigt ist, wobei das mindestens eine Ventilelement ein Abschaltelement umfasst, das dazu ausgelegt ist, in Reaktion auf einen von der Flüssigkeit in dem Kanal auf das Abschaltelement aufgebrachten Druck aus einem geschlossenen Zustand, der den Flüssigkeitsfluss in dem Kanal deaktiviert, in einen offenen Zustand, der den Flüssigkeitsfluss in dem Kanal aktiviert, zu schalten; und
o mindestens ein Sensorelement, das dazu ausgelegt ist, einen Parameter zu messen, der den Druck anzeigt, der auf das Abschaltelement aufgebracht wird, wenn das Abschaltelement aus dem geschlossenen Zustand in den offenen Zustand schaltet,
wobei das Messsystem dazu ausgelegt ist, eine Änderung des Zustands des Abschaltelements aus dem geschlossenen Zustand in den offenen Zustand zu erfassen.

2. Intermittierende Harnkatheterisierungsanordnung nach Anspruch 1, ferner umfassend einen Luftauslass, der dem mindestens einen Ventilelement vorgelagert ist, wobei der Luftauslass dazu ausgelegt ist, keinen Flüssigkeitsdurchlass zu ermöglichen.

3. Intermittierende Harnkatheterisierungsanordnung nach Anspruch 1 oder 2, wobei das mindestens eine Ventilelement dazu ausgelegt und dahingehend steuerbar ist:
o eine Kraft auf das Abschaltelement aufzubringen, um einen Flüssigkeitsdurchfluss in dem Kanal zu deaktivieren, wenn die Signalverarbeitungseinrichtung in Bezug auf den intermittierenden Harnkatheter befestigt ist; und
o die auf das Abschaltelement aufgebrachte Kraft schrittweise zu verringern,
und wobei der Parameter, der den Druck kennzeichnet, der auf das Abschaltelement aufgebracht wird, wenn das Abschaltelement aus dem geschlossenen Zustand in den offenen Zustand schaltet, und von dem mindestens einen Sensorelement messbar ist, die auf das Abschaltelement aufgebrachte Kraft kennzeichnet.

4. Intermittierende Harnkatheterisierungsanordnung nach einem der vorhergehenden Ansprüche, wobei mindestens eins des mindestens einen Sensorelements dazu ausgelegt ist, das Vorhandensein einer dem mindestens einen Ventilelement nachgelagerten Flüssigkeit zu messen.

5. Intermittierende Harnkatheterisierungsanordnung nach einem der vorhergehenden Ansprüche, wobei das Abschaltelement ein ferromagnetisches Ventilelement und mindestens einen Elektromagneten umfasst, wobei der mindestens eine Elektromagnet dazu ausgelegt ist, eine variable Kraft auf das ferromagnetische Ventilelement aufzubringen.

6. Intermittierende Harnkatheterisierungsanordnung nach Anspruch 5, wobei das mindestens eine Sensorelement dazu ausgelegt ist, eine Menge an elektrischem Strom zu messen, der durch den Elektromagneten geleitet wird.

7. Intermittierende Harnkatheterisierungsanordnung nach einem der vorhergehenden Ansprüche, wobei das Messsystem dazu ausgelegt ist, die Änderung des Zustands des Abschaltelements zu erfassen, wenn der Parameter oder ein Derivat davon einen vorbestimmten Schwellenwert übersteigt.

8. Intermittierende Harnkatheterisierungsanordnung nach einem der vorhergehenden Ansprüche, wobei:
- das Ventilelement ferner ein Begrenzungselement umfasst, das dem Abschaltelement vorgelagert ist;
- das Abschaltelement bezogen auf das Begrenzungselement verschiebbar ist;
- das Abschaltelement dazu ausgelegt ist, sich in dem geschlossenen Zustand des Ventilelements dichtend mit dem Begrenzungselement zu verbinden.

9. Intermittierende Harnkatheterisierungsanordnung nach Anspruch 8, wobei:
- das mindestens eine Ventilelement ferner ein dem Begrenzungselement nachgelagertes Halteelement umfasst; wobei das Halteelement dazu ausgelegt ist, das Abschaltelement in dem offenen Zustand des Ventilelements zu halten.

10. Intermittierende Harnkatheterisierungsanordnung nach einem der vorhergehenden Ansprüche, wobei der intermittierende Harnkatheter dazu ausgelegt ist, für einen Zeitraum von maximal 15 Minuten in der Harnröhre des Benutzers zu verbleiben.

11. Intermittierende Harnkatheterisierungsanordnung nach einem der vorhergehenden Ansprüche, wobei mindestens eine Außenseite des einführbaren Abschnitts des Katheterschlauchs eine hydrophile Oberflächenbeschichtung umfasst.

12. Intermittierende Harnkatheterisierungsanordnung nach einem der vorhergehenden Ansprüche, wobei der Katheterschlauch ein einlumiger Schlauch ist, aus dem der Kanal den alleinigen Durchgang zwischen dem distalen Einführende und dem proximalen Auslassende des intermittierenden Harnkatheters bildet.

13. Intermittierende Harnkatheterisierungsanordnung nach einem der vorhergehenden Ansprüche, wobei die Signalverarbeitungseinrichtung dazu ausgelegt ist, eine Fluiddurchflussrate in dem Kanal auf Basis einer von dem mindestens einen Sensorelement durchgeführten Messung zu bestimmen.

14. Intermittierende Harnkatheterisierungsanordnung, umfassend:
- einen intermittierenden Harnkatheter, umfassend:
o einen Katheterschlauch, der einen einführbaren Abschnitt, der zum Einsatz in die Harnröhre eines Benutzers gedacht ist, einen nicht einführbaren Abschnitt, der nicht zum Einsatz in die Harnröhre des Patienten gedacht ist, umfasst; und
o einen Kanal, der sich in Längsrichtung in dem Schlauch erstreckt und mindestens einen Teil eines Fließweges von einem distalen Einführende des Katheters zu einem proximalen Auslassende davon definiert;
- ein Messsystem, das sicher mit dem nicht einführbaren Abschnitt des Katheterschlauchs verbunden oder darin integriert ist, um mindestens einen Druck in dem Kanal und/oder in einem Raum in Verbindung mit dem Kanal zu bestimmen, wobei das Messsystem umfasst:
o eine Signalverarbeitungseinrichtung;
o mindestens ein Ventilelement in Fluidverbindung mit dem Kanal, wobei das mindestens eine Ventilelement ein Abschaltelement umfasst, das dazu ausgelegt ist, in Reaktion auf einen Druck, der durch die Flüssigkeit in dem Kanal auf das Abschaltelement aufgebracht wird, aus einem geschlossenen Zustand, der den Flüssigkeitsdurchfluss in dem Kanal deaktiviert, in einen offenen Zustand, der den Flüssigkeitsdurchfluss in dem Kanal aktiviert, zu schalten; und
o mindestens ein Sensorelement, das dazu ausgelegt ist, einen Parameter zu messen, der den Druck kennzeichnet, der auf das Abschaltelement aufgebracht wird, wenn das Abschaltelement von dem geschlossenen Zustand in den offenen Zustand schaltet,
wobei das Messsystem dazu ausgelegt ist, zu erfassen, wenn das Abschaltelement aus dem geschlossenen Zustand in den offenen Zustand schaltet.

## Revendications

1. Ensemble de cathétérisme urinaire intermittent comprenant :
- un cathéter urinaire intermittent comprenant :
∘ un tube de cathéter comprenant une partie insérable prévue pour une insertion dans l'urètre d'un utilisateur, une partie non insérable non prévue pour une insertion dans l'urètre de l'utilisateur, et une partie de raccordement faisant partie intégrante de la partie non insérable ou montée sur celle-ci ; et
∘ un conduit s'étendant longitudinalement à l'intérieur du tube et définissant au moins une partie d'un chemin d'écoulement depuis une extrémité d'insertion distale du cathéter jusqu'à une extrémité de sortie proximale de celui-ci ;
- un système de mesure configuré pour mesurer au moins une pression dans le conduit et/ou dans un espace en communication avec le conduit, dans lequel le système de mesure comprend :
∘ un dispositif de traitement de signal comprenant un boîtier doté d'un mécanisme d'entrée en prise permettant de fixer de manière amovible le dispositif de traitement de signal par rapport à la partie de raccordement du cathéter ;
∘ au moins un élément de valve en communication fluidique avec le conduit lorsque le dispositif de traitement de signal est solidarisé par rapport au cathéter urinaire intermittent, l'au moins un élément de valve comprenant un élément de coupure configuré pour passer d'un état fermé interdisant l'écoulement de liquide dans le conduit à un état ouvert permettant l'écoulement de liquide dans le conduit en réponse à une pression appliquée à l'élément de coupure par un liquide dans le conduit ; et
∘ au moins un élément capteur configuré pour mesurer un paramètre indiquant la pression appliquée à l'élément de coupure lorsque l'élément de coupure passe de l'état fermé à l'état ouvert,
dans lequel le système de mesure est configuré pour détecter un changement d'état de l'élément de coupure de l'état fermé à l'état ouvert.

2. Ensemble de cathétérisme urinaire intermittent selon la revendication 1, comprenant en outre une sortie d'air en amont de l'au moins un élément de valve, la sortie d'air étant configurée pour ne pas permettre le passage de liquide.

3. Ensemble de cathétérisme urinaire intermittent selon la revendication 1 ou 2, dans lequel l'au moins un élément de valve est configuré et peut être commandé pour :
∘ appliquer une force à l'élément de coupure afin de bloquer un écoulement de liquide dans le conduit lorsque le dispositif de traitement de signal est solidarisé par rapport au cathéter urinaire intermittent ; et
∘ diminuer progressivement la force appliquée à l'élément de coupure,
et dans lequel le paramètre indiquant la pression appliquée à l'élément de coupure lorsque l'élément de coupure passe de l'état fermé à l'état ouvert, mesurable par l'au moins un élément de capteur, est révélateur de la force appliquée à l'élément de coupure.

4. Ensemble de cathétérisme urinaire intermittent selon l'une quelconque des revendications précédentes, dans lequel au moins un de l'au moins un élément capteur est configuré pour mesurer la présence de liquide en aval de l'au moins un élément de valve.

5. Ensemble urinaire intermittent selon l'une quelconque des revendications précédentes, dans lequel l'élément de coupure comprend un élément de valve ferromagnétique et au moins un électroaimant, l'au moins un électroaimant étant configuré pour appliquer une force variable à l'élément de valve ferromagnétique.

6. Ensemble urinaire intermittent selon la revendication 5, dans lequel l'au moins un élément capteur est configuré pour mesurer une quantité de courant électrique conduite à travers l'électroaimant.

7. Ensemble de cathétérisme urinaire intermittent selon l'une quelconque des revendications précédentes, dans lequel le système de mesure est configuré pour détecter ledit changement d'état de l'élément de coupure lorsque le paramètre ou un dérivé de celui-ci dépasse une valeur seuil prédéterminée.

8. Ensemble cathéter urinaire intermittent selon l'une quelconque des revendications précédentes, dans lequel :
- l'élément de valve comprend en outre un élément de restriction en amont de l'élément de coupure ;
- l'élément de coupure est déplaçable par rapport à l'élément de restriction ;
- l'élément de coupure est configuré pour se raccorder de manière étanche à l'élément de restriction dans l'état fermé de l'élément de valve.

9. Ensemble cathéter urinaire intermittent selon la revendication 8, dans lequel :
- l'au moins un élément de valve comprend en outre un élément de retenue en aval de l'élément de restriction ; dans lequel l'élément de retenue est configuré pour retenir l'élément de coupure dans l'état ouvert de l'élément de valve.

10. Ensemble de cathétérisme urinaire intermittent selon l'une quelconque des revendications précédentes, dans lequel le cathéter urinaire intermittent est configuré pour reposer dans l'urètre de l'utilisateur pendant une durée n'excédant pas 15 minutes.

11. Ensemble de cathétérisme urinaire intermittent selon l'une quelconque des revendications précédentes, dans lequel au moins une surface externe de la partie insérable du tube de cathéter comprend un revêtement de surface hydrophile.

12. Ensemble de cathétérisme urinaire intermittent selon l'une quelconque des revendications précédentes, dans lequel le tube de cathéter est un tube à simple lumière, dont le conduit constitue le seul passage entre l'extrémité d'insertion distale et l'extrémité de sortie proximale du cathéter urinaire intermittent.

13. Ensemble de cathétérisme urinaire intermittent selon l'une quelconque des revendications précédentes, dans lequel le dispositif de traitement du signal est configuré pour déterminer un débit de fluide dans le conduit sur la base d'une mesure effectuée par l'au moins un élément capteur.

14. Ensemble de cathétérisme urinaire intermittent comprenant :
- un cathéter urinaire intermittent comprenant :
∘ un tube de cathéter comprenant une partie insérable prévue pour une insertion dans l'urètre d'un utilisateur, une partie non insérable non prévue pour une insertion dans l'urètre de l'utilisateur ; et
∘ un conduit s'étendant longitudinalement à l'intérieur du tube et définissant au moins une partie d'un chemin d'écoulement depuis une extrémité d'insertion distale du cathéter jusqu'à une extrémité de sortie proximale de celui-ci ;
- un système de mesure raccordé ou intégré de façon solidaire à la partie non insérable du tube de cathéter pour déterminer au moins une pression dans le conduit et/ou dans un espace en communication avec le conduit, dans lequel le système de mesure comprend :
∘ un dispositif de traitement de signal ;
∘ au moins un élément de valve en communication fluidique avec le conduit, l'au moins un élément de valve comprenant un élément de coupure configuré pour passer d'un état fermé interdisant l'écoulement de liquide dans le conduit à un état ouvert permettant l'écoulement de liquide dans le conduit en réponse à une pression appliquée à l'élément de coupure par un liquide dans le conduit ; et
∘ au moins un élément capteur configuré pour mesurer un paramètre indiquant la pression appliquée à l'élément de coupure lorsque l'élément de coupure passe de l'état fermé à l'état ouvert,
dans lequel le système de mesure est configuré pour détecter si l'élément de coupure passe de l'état fermé à l'état ouvert.
